# EUROPEAN PATENT SPECIFICATION

(11) **EP 2 204 229 B1**
(45) Date of publication and mention of the grant of the patent: **19.09.2012**
(21) Application number: 08836639.8
(22) Date of filing: 29.09.2008
(51) Int. Cl.: B01F 3/08, A23L 1/30, A61K 9/10, A61K 9/107, A61K 47/10, B01J 13/00

(54) **METHOD OF PRODUCING EMULSION OR DISPERSION AND FOOD, SKIN EXTERNAL PREPARATION AND DRUG CONTAINING EMULSION OR DISPERSION OBTAINED BY THE PRODUCTION METHOD**
VERFAHREN ZUR HERSTELLUNG EINER EMULSION ODER DISPERSION UND NAHRUNGSMITTEL, HAUTPRÄPARAT FÜR DIE ÄUSSERLICHE ANWENDUNG UND ARZNEIMITTEL MIT DER AUF DIESE WEISE HERGESTELLTEN EMULSION ODER DISPERSION
PROCÉDÉ DE FABRICATION D'UNE ÉMULSION OU D'UNE DISPERSION ET D'UNE PRÉPARATION ALIMENTAIRE OU D'UNE PRÉPARATION POUR TRAITEMENT EXTERNE DE LA PEAU, ET MÉDICAMENT CONTENANT L'ÉMULSION OU DISPERSION OBTENUE PAR LEDIT PROCÉDÉ

(30) Priority: 03.10.2007 JP 2007260335
(43) Date of publication of application: 07.07.2010
(73) Proprietor: FUJIFILM Corporation, Tokyo 106-8620 (JP)
(72) Inventor: ARAKAWA, Jun, Ashigarakami-gun Kanagawa 258-8577 (JP); UEYAMA, Tomohide, Minami-Ashigara-shi Kanagawa 250-0123 (JP); NAGASAWA, Hideharu, Minami-Ashigara-shi Kanagawa 250-0123 (JP)
(74) Representative: HOFFMANN EITLE
(86) International application number: PCT/JP2008/067616
(87) International publication number: WO 2009/044694

(56) References cited:
- EP-A1- 1 864 578
- EP-A2- 1 633 463
- EP-A2- 1 905 505
- WO-A1-2005/089928
- JP-A- 2003 113 393
- JP-A- 2004 002 436
- JP-A- 2004 269 492
- JP-A- 2004 333 454
- JP-A- 2007 051 103
- JP-A- 2008 142 588
- JP-A- 2008 189 611
- US-A1- 2003 215 479

## Description

### TECHNICAL FIELD

The present invention relates to a method of producing an emulsion or a dispersion, and a food, a skin external preparation and a drug each of which contains an emulsion or dispersion obtained by the production method.

### BACKGROUND ART

Active ingredients have been extracted from natural animals and plants using water-soluble organic solvents such as alcohols, and have been used in foods and drinks as food additives, or added to cosmetics as functional cosmetics. With the coming of health fad, extraction from various natural materials has widely conducted lately. Extraction of active ingredients from materials that were wasted until recently, such as from banana leaves, leftover portions of citrus and outer skins of onion, is also conducted using alcohols.

Usually, natural active ingredients, which are extracted with water-soluble organic solvents such as alcohols, are thereafter isolated in the form of powders, oils or waxes of the natural active ingredients through the processes of removal of the organic solvents from the extract liquids, concentration and drying, and are used as raw materials of foods or drinks. However, many of the active ingredients do not readily dissolve in water. Therefore, use of the active ingredients in aqueous foods, aqueous drinks, and aqueous cosmetics with superior tactile sensation requires subjecting the active ingredients to processes of emulsification or dispersion after dissolving the active ingredients in other solvents, oils or fats or after melting the active ingredients themselves at high temperatures. As a result, the cost of the processes has been necessarily high. In particular, achievement of effective absorption of the active ingredients requires a decrease of the size of the oil droplets of the active ingredients to 1 µm or less, and a great amount of emulsification energy has been required therefor. Further, there are cases in which the active ingredients deteriorate during the process of once drying the active ingredients and redissolving or melting them. Therefore, a decrease in active ingredient amount and side effects caused by deteriorated ingredients have sometimes been problematic.

In view of the above, the present inventors tried to emulsify and disperse an extract liquid as it is; that is, an aqueous organic solvent containing an active ingredient is emulsified and dispersed, with an emulsifying agent, in an aqueous phase according to usual emulsification methods. Here, the usual emulsification methods are methods using a mechanical force, i.e., methods of dividing an oil droplet by applying a strong shear force from the outside. A most common mechanical force is a high-speed high-shear agitation apparatus. Homomixers, dispermixers, and ultramixers, which are agitation apparatuses of this kind, are commercially available. Other apparatuses that can apply a strong shear force and that are useful for atomization include high-pressure homogenizers, and various kinds thereof are commercially available. Ultrasonic homogenizers, which are relatively energy-efficient dispersing apparatuses, are also used frequently as experiment devices.

When an aqueous organic solvent containing an active ingredient is emulsified and dispersed, using an emulsifying agent, in an aqueous phase according to the usual emulsification methods using an emulsification apparatus such as a high-shear agitation apparatus, a high-pressure homogenizer, or a ultrasonic homogenizer, it is very difficult to emulsify a system containing a large amount of water-soluble organic solvent, and an average particle diameter of 1 µm or less cannot be achieved. The resultant particle diameter distribution is broad, and coarse particles separate out in a short time, causing problems in the preservation properties of foods and cosmetics. When natural active ingredients that are insoluble in water are incorporated into aqueous foods or aqueous cosmetics, it is often requested to finely emulsify and disperse the natural active ingredients, in consideration of digestion, absorption, stability against sedimentation and separation through floating with time, transparency of the appearance, and the like of the active ingredients. In general, an average particle diameter of 200 nm or less is required for fulfilling most of the requests.

Further, another emulsification method of continuously producing microparticles or nano-particles of a natural substance using a micromixer is disclosed in European Patent No. 1180062; granulation of a natural active ingredient is attempted by respectively introducing, into a microflow channel, a particle-forming phase of a natural active ingredient and an aqueous phase whose main component is water, and allowing the respective phases to form layered liquids and periodically mix with each other. However, the average particle diameter of the obtained particles is never below 1000 nm, which is clearly far from a target particle diameter of 200 nm.
EP-A-1 905 505 discloses a method of manufacturing an emulsion by using a nonionic surfactant, an oil agent and water, wherein (i) an emulsification step of forming an emulsion by emulsifying the nonionic surfactant, the oil agent and the water at a phase inversion temperature at which three phases of the nonionic surfactant, the oil agent and the water coexist, and (ii) an emulsion stabilization step of passing the emulsion in a microchannel (72) with an equivalent diameter of ≤ 1 mm to rapidly cool or rapidly heat the emulsion from the phase inversion temperature to a temperature at which the emulsion is stabilized, are conducted in a continuous flow system.
EP-A-1 633 463 (published as WO 2004/103539) relates to a process for making an emulsion, comprising flowing a first liquid through a process microchannel, the process microchannel having a wall with an apertured section; flowing a second liquid through the apertured section into the process microchannel in contact with the first liquid, the first liquid forming a continuous phase, the second liquid forming a discontinuous phase dispersed in the continuous phase.
EP-A-1 864 578 describes a carotenoid-containing emulsion composition comprising carotenoid, at least one water-soluble emulsifier in an aqueous phase, and tocopherol and lecithin in an oil phase.
US 2003/215479 A1 discloses a process for manufacturing hair cosmetic or skin cosmetic products which contain a preparation with hair cosmetic or skin cosmetic ingredients, and one process step includes conducting at least a part of the preparation through an apparatus with microstructured units, which preferably are one or more static micromixers and/or microheat exchangers.
JP-A-2003-113393 relates to a ceramide dispersion capable of being used in various fields by changing the ceramide slightly soluble in water into a water-soluble composition, which ceramide dispersion is formed by dispersing the ceramide (A) in a water-based solvent, wherein an ester (B1) of a C₈₋₁₀-fatty acid of a polyglycerol and another ester (B2) of a C₁₂₋₁₈-fatty acid of the polyglycerol are used in combination.

### DISCLOSURE OF THE INVENTION

### THE PROBLEM TO BE SOLVED BY THE INVENTION

The present invention aims at provision of a fine emulsion or dispersion of a natural ingredient that is extracted from an animal or plant using a water-soluble organic solvent, and that is in a state in which deterioration of the quality of the natural ingredient is extremely small.

### MEANS FOR SOLVING THE PROBLEM

The present invention has been made in view of the above circumstances, and provides 1. A method of producing an emulsion or dispersion, comprising the subsequent steps of:
(i) passing an aqueous solution (w) and a water-soluble organic solvent solution (o) containing a natural ingredient that has been extracted from a natural animal or plant using a water-soluble organic solvent through respective microflow channels each of which has a cross-sectional area at the narrowest portion of 1 µm² to 1 mm²; and
(ii) mixing the solutions (w) and (o) by counter collision.
Preferred embodiments of this method are as defined in the appended dependent claims.
Also, the present invention provides a food, skin external preparation and a drug, each comprising an emulsion or dispersion produced by the present method, wherein the volume average particle diameter of oil droplet particles or dispersed particles in the obtained emulsion or dispersion is 1-50 nm, and wherein the natural ingredient is a functional food ingredient, a skin external preparation ingredient or a pharmaceutical ingredient, respectively.

### BRIEF DESCRIPTION OF THE DRAWINGS

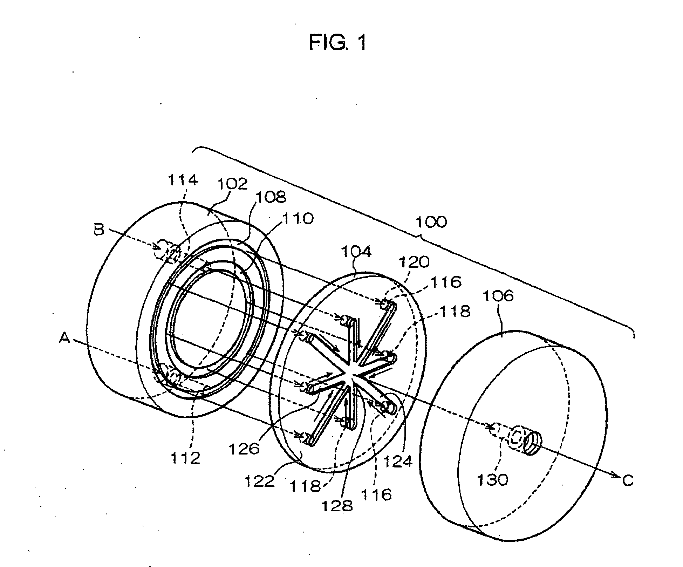

Fig. 1 is an exploded perspective view of a microdevice as an example of a micromixer.

### BEST EMBODIMENT FOR CARRYING OUT THE INVENTION

The method of producing an emulsion or a dispersion according to the invention comprises:
passing an aqueous solution and a water-soluble organic solvent solution containing a natural ingredient that has been extracted from a natural animal or plant using a water-soluble organic solvent through respective microflow channels each of which has a cross-sectional area at the narrowest portion of from I µm² to 1 mm²; and
thereafter mixing the water-soluble organic solvent solution and the aqueous solution by counter collision.

In the production method, a natural ingredient that has been extracted using a water-soluble organic solvent as an extracting liquid is mixed, in a state of a post-extraction solution, i.e., as the water-soluble organic solvent solution, with an aqueous solution. Therefore, it is not necessary to use the natural ingredient in a post-drying state or in a redissolved state after drying. Accordingly, deterioration of the quality of the natural ingredient, such as thermal deterioration due to a drying process, can be minimized. Further, since a water-soluble organic solvent solution containing the natural ingredient, which serves as an oil phase, is mixed with an aqueous solution by counter collision using microchannels, a fine emulsion or a fine dispersion can be obtained.

The term "dispersion" as used in the invention mainly refers to a "dispersion of a solid" or "dispersion of a solid matter" in which a solid that is formed as a result of emulsion, dispersing, or a subsequent process is dispersed in a dispersion medium.
In the invention, the scope of the term "process" not only includes an independent process, but also includes a process that cannot be clearly distinguished from other processes as long as expected actions of the process are achieved.
Expressions indicating numerical ranges in the invention include the upper and lower limit values.

The term "natural ingredient" used in the invention refers to an ingredient that is isolated from an animal or plant by an extraction procedure using an organic solvent. The term "extraction" refers to a chemical isolation procedure, in which a liquid or solid raw material is contacted with a solvent so as to selectively isolate an ingredient that is contained in the raw material and soluble in the solvent, from ingredients that are insoluble or hardly soluble in the solvent. The extraction procedure in the invention is within the scope of the general extraction procedure described above.

The animal or plant raw materials that can be used in the invention are not particularly limited. Supposing that the emulsion or dispersion of the invention is used mainly in health foods or health drinks as the final products, plant materials are preferable compared to animal materials from the viewpoints of odor, taste and healthy image. With respect to the parts of plants, any parts including leaf, stem, peel and fruit can be used as the material. Furthermore, algae, hyphae, yeasts and fungi are preferable as the material for the present invention.

Examples of land plants raw material include *Angelica keiskei, Phaseolus angularis*, *Gynostemma pentaphyllum,* alfalfa, aloe, ginkgo, *Urtica thunbergiana, Azadirachta indica*, Indian gooseberry, *Foeniculum vulgare* (fennel), *Curcuma longa* (turmeric), *Prunus mume, Citrus unshu, Echinacea purpurea, Acanthopanax senticosus, Sambucus nigra* (elder), *Astragalus membranaceus, Plantago asiatica, Onopordum, Hordeum vulgare* (barley), green barley leaf, *Abelmoschu esculentus, Panax ginseng,* oat wheat, *Olea europaea* (olive), *Diospyros kaki, Curcuma zedoaria* (zedoary), *Valerianafauriei, Chamomilla recutita, Paullinia cupana, Glycyrrhiza glabra* (licorice), *Garcinia cambogia, Aloe arborescens, Gymnema sylvestre, Uncaria tomentosa* (cat's claw), *Allium victorialis, Psidium guajava, Lycium chinense, Pueraria lobata, Sasa veitchii, Orthosiphon aristata* (Cumisctin), *Vaccinium macrocarpon* (cranberry), *Citrus grandis* (grapefruit), *Morus alba* (mulberry), *Cinnamomum cassi, Laurus nobilis, Brassica oleracea var. acephala* (kale), *Gentiana lutea,* Kothala himbutu (*Salacia reticulata*), *Coffea arabica* (coffee), *Sesamum indicum* (sesame), *Oryza sativa* (rice), *Triticum vulgare* (wheat), *Amorphophallus konjac* (elephant foot), *Punica granatum* (pomegranate), *Crocus sativus* (saffron crocus), *Crataegus cuneata, Panax notoginseng, Perilla ocymoides [Perilla frutenscens acuta]*, *Pterocarpus santalinus (Dalbergia cochinchinensis), Jasminum officinale* (jasmine), *Betula platyphylla, Zingiber officinale* (ginger), *Equisetum arvense* (field horsetail), *Stevia rebaudiana, Hypericum perforatum* (St. John's wort), *Crataegus oxyacantha, Prunus domestica* (prune), *Taraxacum officinale, Aesculus hippocastanum* (marronier), *Polygonum fagopyrum [Fagopyrum esculentum], Glycine soja* (soybean), *Citrus aurantium* (better orange), *Thymus vulgaris* (thyme), *Tamarindus indica, Allium cepa* (onion), *Aralia elata* Seemann (Japanese angelica tree), chaste tree, *Thea sinensis* (tea), *Cynara scolymus* (artichoke), camellia, *Centella asiatica, Rubus suavissimus, Capsicum annuum, Houttuynia cordata, Eucommia ulmoides, Solanum lycopersicum* (tomato), *Daucus carota sativa* (carrot), *Phoenix dactylifera* (date), *Allium sativum* (garlic), *Serenoa repens* (Saw palmetto), *Tabebuia impetiginosa* (Pau d'arco), *Nelumbo nucifera, Carum petroselinum* (parsley), *Coix lacryma-jobi ma-yuen* (Job's tears), Capsicum annuum cv (paprika), rose, *Fucus vesiculosus, Vaccinium myrtitllus, Eriobotrya japonica* (loquat), *Tussilago farfara* (coltsfoot), *Vitis binefera* (grape), black cohosh, blueberry, propolis, *Carthamus tinctorius* (safflower), *Spinacia oleracea* (spinach), *Peumus boldu, Lepidium meyenii* (maca), macadamia nuts, Manchurian wild rice, pine, *Ilex paraguariensis* (mate), *Tagetes* spp. (marigold), mandarin orange, *Acer nikoense Maximowicz* (Nikko Maple), *Oenothera biennis* (evening primrose), *Melissa officinalis* (melissa), *Melilotus officinalis Lam, Corchorus olitorius L.* (Mulukhiyya), yucca, *Artemisia princeps* (mugwort), *Apocynum venetum L.* (Luobuma), *Lavandula angustifolia* (lavender), *Pyrus malus* (apple), *Litchi chinensis* (lychee), *Citrus medica lemonum* (lemon), *Rosmarinus offisinalis* (rosemary), *Walteria indica*, moss plants, fern plants and the like, but the invention is not limited thereto.

With respect to algae, all photosynthetic algae generating oxygen can be used. Examples of the algae include cyanobacteria, Prochlorophyta, Glaucophyceae, Rhodophyceae, Prasinophyceae, Ulvophyceae, Chlorophyceae, trebouxiophyceae, Charophyceae, Cryptophyceae, Chlorarachniophyceae, Euglenophyceae, Dinophyceae, Chrysophyceae, Raphidophyceae, Eustigmatophyceae, Xanthophyceae, Phaeophyceae, Cacillariophyceae, Dictyochophyceae, Pelagophyceae, Haptophyceae and the like. Among these algae, spirulina belonging to Cyanophyceae; Haematococcus belonging to Chlorophyceae; and Nemacystus, Laminaria, and Undaria (seaweed) belonging to Phyaeophyceae are particularly important materials.

Various examples are included in hyphae, yeasts and fungi. Examples thereof include Agaricus, yeasts, *Lentinus edodes (Berk.) Sing* (shiitake), *Agaricus bisporus* (champignon), *Cordyceps sinensis* (Cordyceps), *Bacillus subtilis* (Hay bacillus), Bifidobacteria, *Monascus purpureus* (Red yeast rice), *Tremella fuciformis, Grifola frondosa* (Gray-maitake), bisporus (common mushroom), *Phellinus linteus, Hericium erinaceus, Ganoderma lucidum. Karst* (Reishi) and the like, but the invention is not limited thereto.

Various ingredients over a broad range can be extracted from these natural materials. Typical examples thereof include lipid ingredients contained in animals and plants. Examples of the lipid ingredients include fatty acids, glycerides, complex lipids, terpenoids, steroids, prostaglandins and the like.
Among these lipid ingredients, examples of the active ingredient include linoleic acid, γ-linolenic acid, arachidonic acid, α-linolenic acid, eicosapentaenoic acid, docosahexaenoic acid, γ-aminobutyric acid, thioctic acid and the like. Examples of the glycerides include monoacylglycerol, diacylglycerol and triacylglycerol. Examples of the complex lipids include phospholipids such as phosphatidic acid, phosphatidylcholine, phosphatidylethanolamine, phosphatidylserine and phosphatidylinositol; sphingo lipids such as sphingosine and sphingomyelin; and glycolipids such as monogalactosyl glyceride and glucosyl ceramide.

Examples of the active ingredients of terpenoids include monoterpenes such as linalool, citronellal, myrcene, limonene, pinene, menthol, cineol, camphor, longifolene, cedrol and caryophyllene; diterpenoids such as phytol, abietic acid, kaurene and gibberellin; triterpenoids such as squalene and dammarenediol; tetraterpenoids typified by carotenoid pigments; and polyterpenoids such as gutta-percha, vitamin K2, ubiquinone and vitamin K1. Among these substances, carotenoid pigments may attract particular attention owing to the antioxidative activity thereof. Typical examples of the carotenoid pigments include astaxanthin, lycopene, zeaxanthin, lutein, capsanthin, fucoxyanthin, α-carotene and β-carotene.

Examples of the active ingredients of the steroids include sterols such as squalene, cholesterol, ergosterol, stigmasterol, dehydrocholesterol, cholecalciferol (vitamin D3) and 25-hydroxy vitamin D3; sex hormones such as testosterone, androsterone, progesterone, estrogen and estradiol; adrenocortical hormones such as cortisone and dexamethasone; cardiac glycosides such as digitoxigenin, digoxigenin and gitoxigenin; steroid sapogenins such as diosgenin and cortisone; and bile acids such as cholic acid and deoxycholic acid.

One of important natural ingredients other than lipid ingredients is polyphenol. Polyphenol is a generic name for plant ingredients having two or more phenolic hydroxyl groups within a molecule. Polyphenol constitutes a plant pigment and a bitter ingredient formed by photosynthesis, and has particularly excellent antioxidation activity. Examples of polyphenols include flavonoids, chlorogenic acids, gallic acids, ellagic acids, lignans, curcumins and coumarins. Examples of flavonoids include isoflavones such as genistein and daizen; flavonols such as quercetin, kaempferol, myricetin and rutin; flavanones such as hesperidin and naringin; anthocyanins such as cyanidin and delphinidin; flavanols such as epicatechin, epigallocatechin, epicatechin gallate and theaflavin; and flavones such as chrysin, apigenin and luteolin. Many of polyphenols have attracted public attention as ingredients of health foods and cosmetics, and examples of such polyphenols include galangin, fisetin, chalcone, puerarin and resveratrol.

Other natural ingredients include alkaloids, which are particularly useful in the medicinal field. Examples of the alkaloids include aconitine, atropine, ephedrine, caffeine, capsaicin, quinine, curare, cocaine, colchicine, scopolamine, strychnine, solanine, taxine, theophylline, dopamine, nicotine, vinca, berberine, morphine, lycorine and the like.

Furthermore, other natural ingredients include shogaol and gingerol extracted from ginger, allyl isothiocyanate that is contained in horseradish or mustard, and allicin, alliin and scordinine contained in garlic, and the like.

The water-soluble organic solvent in the present invention, which serves as an oil phase containing natural component, is used for mixing with a below described aqueous solution. The water-soluble organic solvent is also a main component of the extracting liquid with which the natural component is extracted. That is, in the invention, the natural integrant is used to be mixed with the aqueous solution in a state where the natural integrant is extracted with the extracting liquid containing the water-soluble organic solvent as the main component.
The term "water-soluble organic solvent" to be used in the invention refers to an organic solvent which has a solubility in water (at 25°C) of 10% by mass or more. From the viewpoint of the stability of the obtained emulsion or dispersion, the solubility in water is preferably 30% by mass or more, and more preferably 50% by mass or more.

The water-soluble organic solvent may be used singly or as a mixed solvent of plural water-soluble organic solvents. Further, the water-soluble organic solvent may be used as a mixture with water. When the water-soluble organic solvent is used as the mixture with water, the content of the water-soluble organic solvent in the mixture is preferably 50% by volume or more, and more preferably 70% by volume or more.

Examples of the water-soluble organic solvent include methanol, ethanol, 1-propanol, 2-propanol, 2-butanol, acetone, tetrahydrofuran, acetonitrile, methyl ethyl ketone, dipropylene glycol monomethyl ether, methyl acetate, methyl acetoacetate, N-methylpyrrolidone, dimethyl sulfoxide, ethylene glycol, 1,3-butanediol, 1.4-butanediol, propylene glycol, diethylene glycol, triethylene glycol and the like, and mixtures thereof. Among these, ethanol, propylene glycol and acetone are preferable, and ethanol and a mixture of ethanol with water are more preferable when the application thereof is limited to foods.

The method of extracting a natural ingredient from the above-described animal and plant raw materials using a water-soluble organic solvent may be a conventional method. Since solid raw materials obtained by finely crushing raw materials such as dried plants are often used, solid-liquid extraction apparatuses are mainly used. In experiments, a simple method of performing extraction procedures in a beaker or the like while stirring and maintaining a constant temperature, or a soxhlet extractor or the like, may be used. Examples of solid-liquid extraction apparatuses that are used industrially include permeation through-flow extractors, and examples thereof include a battery extractor, which is a batch-type extractor, and a Bollman extractor, a Rotcel extractor, a Lurgi extractor, and a Kennedy extractor, which are continuous-type extractors. As a solid-dispersing apparatus, a Pachuca tank extractor, a bonotto extractor, or the like may be used.

In the invention, it is preferable that the animal, plant, alga, yeast, bacterium, or the like to be used for extraction is sufficiently washed with water, and then directly, or after drying, subjected to a pre-treatment which is a physical crushing treatment such as a ball milling, frost shattering, compression crushing or ultrasonic treatment, an enzymatic treatment, or the like. When extraction from a wet material is performed using an organic solvent, incorporation of contaminants is severe, and extraction ratio is decreased in every case. Therefore, solvent extraction from dried microbial body is industrially preferable.

Extraction employed in the invention is conducted representatively at a temperature that is sufficiently high for causing significant extraction and that is lower than a temperature at which the properties of the active ingredient to be extracted is adversely affected (or a temperature higher than a reasonable reflux temperature of the selected extraction solvent). The extraction temperature is generally in a range of from 0°C to 300°C, but may be a temperature that is higher or lower than this range. In general, the extraction temperature depends on the boiling or freezing characteristics of the extraction solvent or of the solution. Basically, a higher temperature results in a higher extraction speed. Therefore, a moderate temperature is preferable for achieving an economical extraction speed. A usual extraction temperature is from 20°C to 68°C, and the extraction temperature is preferably from about 20°C to about 50°C.
Selection of the extraction solvent depends on, representatively, factors such as chemical properties of the active ingredient to be extracted, dissolution properties, boiling point, and freezing point. The extraction solvent may be selected preferably from the water-soluble organic solvents described above. An alcohol, such as ethanol or n-propyl alcohol, is a favorable extraction solvent, regardless of whether the alcohol is undiluted or diluted with water. Ethanol is a particularly preferable extraction solvent.

When the extraction solvent to be used is, for example, ethanol, extraction is conducted for from about 30 minutes to about 2 hours using the extraction solvent in an amount that is from 3 times to 100 times greater (by weight) than that of the raw material to be extracted. After the active ingredient is eluted into the solvent, extraction residue is removed by filtration, as a result of which an extract liquid is obtained. Thereafter, the extract liquid is subjected to one or more treatments such as dilution, concentration, or purification according to a common method, whereby an ethanol solution of the active ingredient according to the invention is obtained.

The residue of the animal or plant raw material remaining after extraction of the natural ingredient can be removed using a method whereby a solid component and a liquid component of the immersion liquid can be separated. The method may be, for example, any of filtration, centrifugal separation, sedimentation by being left to stand still, or a combination thereof. When conducting filtration, the filtration manner is not limited. For example, the filtration may be performed by natural dropping, or may be performed under pressure control such as vacuum filtration. The filter portion may be any of a paper filter, a membrane filter, a cloth filter, a charcoal filter, a hollow fiber membrane filter, a micro filter, a CELITE filter, a diatomite filter, or a combination thereof. The filter portion may have either a single layer or multiple layers. When using a multilayer filter, the multilayer filter may be composed of plural layers that may the same or different. Further, the layers of the multilayer filter are not required to be stacked vertically or horizontally, as long as the layers are arranged such that the liquid component is allowed to go through the respective layers. For example, when plural columns filled with filter component(s) are connected via tubes so as to enable filtration, the respective layers may be separated from each other.

When conducting centrifugal separation, the manner of separating the solid component and the liquid component is not limited. For example, the filtration manner may be a manner in which an immersion liquid is introduced into a porous tube, the porous tube is centrifuged in a centrifuge, and an extract liquid discharged from the pores is collected, or may be a manner in which an immersion liquid is introduced into an imperforate tube and an extract liquid as a supernatant is collected after centrifugation. The gravitational acceleration of the centrifugation (G) is not particularly limited as long as the liquid component and the solid component can be separated to a certain degree.

When the extract liquid of the natural ingredient of the invention is contacted with at least one selected from activated carbon, acid clay, or activated earth before removing insoluble solid matter from the extract liquid by filtration or other methods, removal of the insoluble solid matter becomes extremely easy. For example, when removing the insoluble solid matter by filtration, the number of filtration stages can be minimized, for example to once. The activated carbon to be used may be selected, without particular restrictions, from activated carbons generally utilized in industries. Examples of activated carbons that may be used include commercial products such as ZN-50 (manufactured by Hokuetsu Carbon Industry Co., Ltd.), KURAREY COAL GLC, KURAREY COAL PK-D, and KURAREY COAL PW-D (manufactured by Kurarey Chemical Co., Ltd.), and SHIRASAGI AW50, SHIRASAGI A, SHIRASAGHI M, and SHIRASAGI C (manufactured by Takeda Pharmaceutical Company Limited). The pore volume of the activated carbon is preferably from 0.01 to 0.8 mL/g, and the specific surface area thereof is preferably from 800 to 1300 m²/g. The amount of the activated carbon is preferably from 0.5 to 5 parts by mass relative to 100 parts by mass of the ethanol solution.

The content of the natural ingredient in the water-soluble organic solvent solution in the invention varies with the types of the raw material and the natural ingredient, and may generally be from 0.0 1 % by mass to 30% by mass, preferably from 0.1 % by mass to 10% by mass, relative to the total mass of the water-soluble organic solvent solution, from the viewpoints of the efficiency of the production of an emulsion or dispersion and the stability of the emulsion or dispersion.
The water-soluble organic solvent solution may include, in addition to the natural ingredient, other ingredients as necessary. Examples of the other ingredients include oil ingredients such as hydrogenated oils and fats and silicone oils, and surface active compounds such as nonionic surfactants, ionic surfactants, synthetic phospholipids, and synthetic ceramides.

The aqueous solution in the invention, i.e., the aqueous phase, is an aqueous solution whose main component is water.
The aqueous solution may include one or more of a nonionic surfactant, an ionic surfactant, a water-soluble salt, a saccharide, a polysaccharide, a protein, a pH adjuster, an antioxidant, a preservative, a colorant, a perfume, or the like, such as those described below.

Examples of the ionic surfactant include an alkylsulfonate, an alkylsulfate, a monoalkylphosphate, a fatty acid salt, and lecithin. Examples of the salt include sodium chloride, sodium citrate, and sodium ascorbate. Examples of the saccharide include glucose, fructose, sucrose, arabinose, cellobiose, lactose, maltose, and trehalose. Examples of the polysaccharide include maltodextrin, oligosaccharides, inulin, gum arabic, and chitosan. Examples of the protein include various amino acids, oligopeptides, gelatin, water-soluble collagen, casein, and cyclodextrin.

The pH adjuster to be used may be a base such as sodium hydroxide, an acid such as hydrochloric acid, or a buffer solution such as a phosphate buffer solution or a citrate buffer solution. Examples of the antioxidant include ascorbic acid, an ascorbic acid derivative, and a citric acid monoglyceride.

The amount of the additives to be added to the aqueous phase may be 20% by mass or less, preferably 10% by mass or less, relative to the total mass of the aqueous phase, with the view to finely emulsifying and dispersing. A small amount of water-soluble organic solvent may be added to the aqueous phase in advance, as necessary. The amount of the water-soluble organic solvent to be added in this case is 20% by mass or less, preferably 10% by mass or less, relative to the total mass of the aqueous phase, from the viewpoint of the stability of the emulsion or dispersion.

### [Nonionic Surfactant]

The nonionic surfactant in the invention is preferably a nonionic surfactant having an HLB value of from 10 to 16 with the view to improving the dispersibility, and the HLB value is more preferably from 12 to 16 from the viewpoint of the stability of the emulsion.
The nonionic surfactant may be contained in either one of the organic solvent phase or the aqueous phase, or in both of the organic solvent phase and the aqueous phase. It is preferable that at least one nonionic surfactant is added to the organic solvent phase. In this case, the production method according to the invention may further include a process of adding a nonionic surfactant to the water-soluble organic solvent solution and uniformizing the solution, before the water-soluble organic solvent solution is mixed with the aqueous solution.

At least one of the nonionic surfactants that can be used in the invention is preferably a nonionic surfactant capable of dissolving in an organic solvent, from the viewpoint of decreasing the dispersion particle diameter. The nonionic surfactant soluble in an organic solvent may be selected, without particular restrictions, from nonionic surfactants that are soluble in an organic solvent.

The HLB used herein represents hydrophilic-hydrophobic balance generally used in the field of surfactants. It can be calculated by using generally used calculation formula such as Kawakami's equation. In the invention, the following Kawakami's equation is employed.

HLB = 7 + 11.7 log (Mw/Mo)
In the above equation, Mw represents the molecular weight of a hydrophilic group, and Mo represents the molecular weight of a hydrophobic group.

Alternatively, numerical values of HLB listed in manufacture's catalogs may be used. As is apparent from the above equation, a nonionic surfactant with any HLB value can be obtained by utilizing the additive properties of HLB.

Examples of nonionic surfactants that may suitably be used in the invention include (mono, di, or tri)glycerol fatty acid esters, monoglycerol organic acid esters, polyglycerol fatty acid esters, propylene glycol fatty acid esters, polyglycerol condensed ricinoleic acid esters, sorbitan fatty acid esters, and sucrose fatty acid esters. Among the above, a more preferable nonionic surfactant in the invention is a polyglycerol fatty acid ester, sucrose fatty acid ester, or a combination thereof, from the viewpoint of improving the stability of the dispersion. Any one of the nonionic surfactants may be used singly, or two or more thereof may be used in combination at an arbitrary ratio.
The nonionic surfactant is not necessarily a highly purified product obtained by distillation or the like, and may be a reaction mixture.

Examples of the polyglycerol fatty acid esters include an ester of a polyglycerol having an average degree of polymerization of 4 or more, preferably 6 to 10, and a fatty acid having 8 to 18 carbon atoms (for example, caprylic acid, capric acid, lauric acid, myristic acid, palmitic acid, stearic acid, oleic acid, and linoleic acid).
Preferable examples of the polyglycerol fatty acid ester include hexaglyceryl monopalmitate, hexaglyceryl monomyristate, hexaglyceryl monolaurate, decaglyceryl monooleate, decaglyceryl monostearate, decaglyceryl monopalmitate, decaglyceryl monomyristate, and decaglyceryl monolaurate.
These polyglycerol fatty acid ester can be used singly or as a mixture thereof.
Examples of the commercially available product of polyglycerol fatty acid esters include NIKKOL HEXAGLYN 1-L, NIKKOL HEXAGLYN 1-M, NIKKOL HEXAGLYN 1-L, NIKKOL DECAGLYN 1-M, NIKKOL DECAGLYN 1-SV, NIKKOL DECAGLYN 1-50SV, NIKKOL DECAGLYN 1-ISV, NIKKOL DECAGLYN 1-O, NIKKOL DECAGLYN 1-OV and NIKKOL DECAGLYN 1-LN (manufactured by Nikko Chemicals Co., Ltd.); RYOTO polyglyester L-10D, L-7D, M-10D, M-7D, P-8D, S-28D, S-24D, SWA-20D, SWA-15D, SWA-10D and O-15D (manufactured by Mitsubishi-Kagaku Foods Corporation); and Poem J-0381V and Poem J-0021 (manufactured by Riken Vitamin Co., Ltd.).

The sucrose fatty acid ester to be used in the invention is preferably one having 12 or more, more preferably from 12 to 20 carbon atoms in the fatty acid moiety.
Preferable examples of the sucrose fatty acid ester include sucrose dioleate, sucrose distearate, sucrose monopalmitate, sucrose monomyristate and sucrose monolaurate.
In the invention, these sucrose fatty acid esters may be used singly or may used as a mixture thereof.
Examples of the commercially available product of sucrose fatty acid esters include, but are not limited to, RYOTO sugar ester S-1170, S-1170S, S-1570, S-1670, P-1570, P-1670, M-1695, O-1570, OWA-1570, L-1695, and LWA-1570 (manufactured by Mitsubishi-Kagaku Foods Corporation); and DK ester F140, DK ester F160 and DK ester SS (manufactured by DAI-ICHI KOGYO SEIYAKU CO., LTD.).

In the invention, lecithin may be used in combination with the water-soluble nonionic surfactant described above. The lecithin used in the invention contains a glycerol skeleton, a fatty acid residue, and a phosphoric acid residue as essential constituent components, and one or more of a base, a polyhydric alcohol, or the like are bonded thereto. The lecithin used in the invention is also referred to as "phospholipid".
Since lecithin has a hydrophilic group and a hydrophobic group within a molecule, lecithin has widely been used as an emulsifying agent in the fields of foods, drugs, and cosmetics.

Industrially, products having a lecithin purity of 60% or higher are used as lecithins, and can be used in the invention. However, from the viewpoints of formation of fine oil droplets and stability of the functional oil component, lecithins that are generally called high-purity lecithins are preferable, and the lecithin purity of the high-purity lecithins is 80% by mass or higher, and more preferably 90% by mass or higher.

Examples of lecithins include various known lecithins that are extracted and isolated from living bodies of plants, animals, and microorganisms.
Specific examples of such lecithins include various lecithins derived from, for example, plants such as soybean, Indian corn, peanut, rapeseed, and wheat, animals such as egg yolk and cow, and microorganisms such as Escherichia coli.
Examples of the compound names of the lecithins include glycerolecithins such as phosphatidic acid, phosphatidylglycerol, phosphatidylinositol, phosphatidylethanolamine, phosphatidyl methylethanolamine, phosphatidylcholine, phosphatidylserine, bisphosphatidic acid, and diphosphatidyl glycerol (cardiolipin), and sphingolecithins such as sphingomyelin, and combinations thereof.
In the invention, other than the high-purity lecithins, the following lecithins may be used: hydrogenated lecithins, enzymatically decomposed lecithins, enzymatically decomposed hydrogenated lecithins, hydroxy lecithins, and combinations thereof. Any of these lecithins, which may be used in the invention, may be used singly, or in the form of a mixture of two or more kinds thereof.

The amount of the nonionic surfactants to be added is preferably from 0.1 to 50% by mass, more preferably from 0.5 to 20% by mass, and still more preferably from 1 to 15% by mass, relative to the total mass of the emulsion or dispersion.
The amount of the nonionic surfactants to be added is preferably from 10 to 1000% by mass, and more preferably from 50 to 500% by mass, relative to the natural ingredient.
A nonionic surfactant addition amount of 0.1% by mass or more makes it easy to obtain a dispersion having a fine particle diameter, improves the stability of the obtained dispersion, and thus is preferable. A nonionic surfactant addition amount of 50% by mass or less is preferable since problems such as severe foaming of the dispersion hardly occur at such an addition amount.

In addition to the water-soluble organic solvent used also as an extracting liquid, one or more other water-soluble organic solvents may be mixed into the water-soluble organic solvent solution. In addition to the water-soluble organic solvent used also as an extracting liquid, water may be added. The mixing ratio of the water-soluble organic solvents and water added afterwards is preferably 50% by volume or lower, and more preferably 30% by volume or lower, relative to the total mass of the water-soluble organic solvent solution after mixing therewith, from the viewpoint of forming finer emulsion particles.

### (Micromixer)

In the invention, a most suitable device for passing the water-soluble organic solvent solution (hereinafter simply referred to as "oil phase" or "water-soluble organic solvent phase" in some cases) and the aqueous solution (hereinafter simply referred to as "aqueous phase" in some cases) through microchannels of which narrowest portion has a cross-sectional area of from 1 µm² to 1 mm² and thereafter mixing them by counter collision is counter-collision micromixer. The micromixer is generally a device which mixes two different liquids in a microspace, one of the liquids is an organic solvent phase containing a functional oil component, and the other liquid is an aqueous phase which is an aqueous solution.
The inventors have found that application of a micromixer to preparation of an emulsion having a small particle diameter, which is a microchemical process, exhibits relatively low energy consumption, generates less heat, and enables provision of an emulsion or dispersion that has substantially uniform particle diameters and has excellent storage stability compared to usual agitation emulsification dispersing methods or high-pressure homogenizer emulsification dispersing methods. The inventors have also clarified that application of a micromixer is a most suitable method for emulsification when a natural ingredient that is susceptible to thermal deterioration is contained.

In summary, an emulsification or dispersing method using a micromixer includes distributing the respective aqueous oil phases to microspaces, and contacting or colliding them in the respective microspaces. This method is clearly different from a membrane emulsification method or a microchannel emulsification method, in which only one phase is distributed to microspaces and the other phase is supplied as a bulk. Indeed, when only one phase is distributed to microspaces, the effect of the invention cannot be obtained. Known micromixers feature various different structures. In terms of flow and mixing in microchannels, two kinds of method can be exemplified: a method in which mixing is achieved while maintaining a laminar flow, and a method in which mixing is achieved while disturbing a flow, that is, in a turbulent flow. In the method in which mixing is achieved while maintaining a laminar flow, the efficiency of mixing is improved by adjusting the depth dimension of flow channels to be larger than the width dimension of the flow channels, increasing the interface area between the two different liquids as far as possible, and reducing the thickness of both layers. Alternatively, a method in which two different liquids are alternately flowed in a multilayer flow via a multiply-segmented inlet for the liquids has also been proposed.

Meanwhile, a method in which mixing is achieved in a turbulent flow is generally a method in which respective liquids are flowed at a relatively high speed by distributing them to narrow flow channels. A method in which one liquid is sprayed into the other liquid that has been introduced into microspaces using an arrayed micro-nozzle has also been proposed. Furthermore, a particularly excellent mixing effect can be obtained in a method in which liquids flowing at a high speed are forcibly contacted with each other using various means.
The former method using a laminar flow generally produces particles that have large diameter but relatively uniform particle diameter distribution. In the latter method using a turbulent flow, there is a possibility that a very fine emulsion is obtained, and thus the method using a turbulent flow is preferable in many cases in view of stability and transparency. Representative methods using a turbulent flow include a method using a slit-interdigital-type micromixer and a method using a collision-type micromixer. A slit-interdigital-type micromixer, as typified by a mixer manufactured by IMM Gmbh, has a structure in which two interdigital flow channels are faced and arranged such that one channel enters between the two other channels. Although a turbulent flow is caused when the width between the slits is sufficiently small, an organic solvent phase and an aqueous phase flow in the same direction in a parallel flow without colliding with each other after merging. Therefore, the forcible contacting effect in a slit-interdigital micromixer has not been sufficient compared to a collision-type micromixer.

A collision-type micromixer, represented by a KM mixer, has a structure in which forcible contact is conducted by utilizing kinetic energy. Specifically, collision-type micromixers include a central collision-type micromixer disclosed by Nagasawa et al. ("H. Nagasawa et al., Chem. Eng. Technol., 28, No.3 , 324-330 (2005)"; JP-A No. 2005-288254). It has become evident that an extremely fine emulsion or dispersion can be easily formed in the method of countercurrently colliding an aqueous phase and an organic solvent phase, since mixing time is extremely short and an oil phase droplet is instantly formed.

In the invention, when emulsification is performed by micro-mixing with a collision-type micromixer, it is required that the temperature at emulsification (emulsification temperature) is such that micro-mixing is performed at a temperature of the aforementioned separate microspaces of the micromixer (a temperature at micro-mixing part of the micromixer) of 80°C or lower, preferably from 0°C to 80°C and particularly preferably from 5°C to 75°C, from the viewpoint of uniformity of the particle diameter in the resulting emulsion. An emulsification temperature of 0°C or higher is preferable since a main component of the dispersing medium is water and, therefore, the emulsification temperature can be managed. The temperature of the microspaces of the micromixer is preferably maintained at 100°C or lower. When the temperature is maintained at 100°C or lower, management of the retained temperature may be easily controlled, and the micro-bumping phenomenon which adversely influences the emulsification performance may be prevented. It is further preferable that the temperature is controlled to be maintained at a temperature of 80°C or lower.

The temperature at which the oil phase (water-soluble organic solvent phase) that is distributed to the microspaces of the micromixer is maintained, the temperature at which the aqueous phase that is distributed to the microspaces of the micromixer is maintained, and the temperature at which the microspaces of the micromixer are maintained vary depending on the components contained in the aqueous phase and the organic solvent phase, and are each independently preferably from 0°C to 80°C, particularly preferably from 5°C to 75°C. When the oil phase includes a water-soluble organic solvent, the temperature at which the oil phase that is distributed to the microspaces of the micromixer is maintained, the temperature at which the aqueous phase that is distributed to the microspaces of the micromixer is maintained, and the temperature at which the microspaces of the micromixer are maintained are each independently preferably from 0°C to 50°C, and particularly preferably from 5°C to 25°C. Although the temperature at which the microspaces of the micromixer are maintained, the temperature at which the oil phase and the aqueous phase distributed to the microspaces of the micromixer are maintained, and the temperature at which the oil phase and the aqueous before distributed to the microspaces of the micromixer are maintained (i.e., the temperature at which the oil phase supply tank and the aqueous phase supply tank are maintained) may be different from one another, they are preferably the same temperature in the point of stability of mixing.

In the invention, it is particularly preferable that the temperatures at which the aqueous phase and the organic solvent phase that are distributed to the microspaces of the micromixer are maintained before and after distribution, the temperature at which the microspaces of the micromixer are maintained, and the temperature at which the aforementioned separate microspaces of the micromixer are maintained, are adjusted to a temperature higher than ambient temperature, and an oil-in-water emulsion obtained by the micromixer after micro-mixing and emulsification is cooled to ambient temperature, after collection.

The cross-sectional area of the narrowest part of the microspaces (flow channels) of the micromixer in the invention is from 1 µm² to 1 mm² and preferably from 500 µm² to 50,000 µm², from the viewpoints of decreasing the emulsion particle diameter and narrowing the particle diameter distribution.
The cross-sectional area of the narrowest part of the microspaces (flow channels) for the aqueous phase of the micromixer in the invention is particularly preferably from 1,000 µm² to 50,000 µm² from the viewpoint of stability of mixing.
The cross-sectional area of the narrowest portion of the microspaces (flow channels) for the oil phase of the micromixer is particularly preferably from 500 µm² to 20,000 µm² from the viewpoints of decreasing the emulsion particle diameter and narrowing the particle diameter distribution.
In the present invention, when emulsifying and dispersing with the micromixer, the flow rate of the oil phase and the aqueous phase in emulsifying and dispersing varies depending on the micromixer used and the flow rate of the aqueous phase is preferably from 10 ml/min to 500 ml/min, more preferably from 20 ml/min to 400 ml/min and particularly preferably from 50 ml/min to 300 ml/min, from the viewpoints of decreasing the emulsion particle diameter and narrowing the particle diameter distribution.
The flow rate of the oil phase is preferably from 1 ml/min to 70 ml/min, more preferably from 3 ml/min to 60 ml/min and particularly preferably from 7 ml/min to 40 ml/min, from the viewpoints of decreasing the emulsion particle diameter and stability of mixing.

The value obtained by dividing flow rates of both phases by the cross-sectional area of a microchannel, that is, the ratio (Vo/Vw) of flow speeds of both phases is preferably in the range from 0.05 to 5 from the viewpoints of forming finer emulsion particles and design of the micromixer. Here, Vo represents the flow speed of an organic solvent phase containing a water-insoluble natural integrant, and Vw represents the flow speed of an aqueous phase. Furthermore, it is most preferable that the range of the ratio (Vo/Vw) of the flow speed is from 0.1 to 3 from the viewpoint of forming further finer emulsion particles.

In addition, the liquid sending pressure of the aqueous phase is preferably from 50 KPa to 5000 KPa, more preferably from 100 KPa to 2000 KPa, and particularly preferably from 200 KPa to 1000 KPa; and the liquid sending pressure of the oil phase is preferably from 10 KPa to 1000 KPa, more preferably from 20 KPa to 500 KPa, and particularly preferably from 40 KPa to 200 KPa. The liquid sending pressure of the aqueous phase of from 50 KPa to 5000 KPa is preferable since the stable solution sending flow rate tends to be maintained. The liquid sending pressure of the oil phase of from 10 KPa to 1000 KPa is preferable, since the uniform mixing property tends to be obtained.
In the invention, the flow rate, the solution sending pressure and the maintained temperature are more preferably a combination of respective preferably examples.

Next, a pathway from introduction of the aqueous phase and the oil phase into the micromixer until discharge thereof as an O/W emulsion is explained using the example of a microdevice (Fig. 1) as one example of the micromixer in the invention.
As shown in Fig. 1, a microdevice 100 consists of supply element 102, a confluence element 104 and a discharge element 106, each having a cylindrical form.
On a surface of the supply element 102 facing the confluence element 104, annular channels 108 and 110 each having rectangular cross-sectional shape are arranged in a concentric pattern as channels for the oil phase or the aqueous phase of the invention. In the supply element 102, bores 112 and 114 are formed leading to each annular channel, penetrating in a thickness (or height) direction.
In the confluence element 104, bores 116 are formed penetrating in a thickness direction. These bores 116 are arranged such that when the elements are fastened to one another to form the microdevice 100, ends 120 of the bores 116 located on a surface of the confluence element 104 facing the supply element 102 open into the annular channel 108. In an embodiment shown in the drawing, four bores 116 are formed, and they are arranged equiangularly to the annular channel 108.

In the confluence element 104, bores 118 penetrating in a thickness direction are formed similarly to the bores 116. The bores 118 are formed such that they open into the annular channel 110, as well as the bores 116. The bores 118 are arranged equiangularly to the annular channel 110, and the bores 116 and the bores 118 are alternately-arranged.
On a surface 122 of the confluent element 104 facing the discharge element 106, the microchannels 124 and 126 are formed. One end of each of these microchannels 124 or 126 is an opening part of the bores 116 or 118, and the other end is a central part 128 of the surface 122. All microchannels extend from bores towards this central part 128, and converge at a center. A cross-sectional shape of the microchannels may be, for example, a rectangular shape.

In the discharge element 106, a bore 130 is formed passing a center thereof and penetrating in a thickness direction. Therefore, this bore is opened into the central part 128 of the confluence element 104 at one end, and is opened to the outside of the microdevice at the other end.
In the present microdevice 100, fluids A and B supplied from the outside of the microdevice 100 at ends of bores 112 and 114 are flown into annular channels 108 and 110 via bores 112 and 114, respectively.

The annular channel 108 and the bores 116 are communicated, and the fluid A flown into the annular channel 108 enters microchannels 124 via the bores 116. In addition, the annular channel 110 and the bores 118 are communicated, and the fluid B flown into the annular channel 110 enters microchannels 126 via the bores 118. Fluids A and B are flown into microchannels 124 and 126, respectively, and are flown towards a central part 128, and are converged.
The converged fluids are discharged as a stream C to the outside of the microdevice via the bore 130.

The following variations of the microdevice 100 may be used.
Annular channel 108: cross-sectional shape/width/depth/diameter: rectangular shape/1.5 mm/1.5 mm/25 mm
Annular channel 110: cross-sectional shape/width/depth/diameter: rectangular shape/1.5 mm/1.5 mm/20 mm
Bore 112: diameter/length: 1.5 mm/10 mm (circular cross-section)
Bore 114: diameter/length: 1.5 mm/10 mm (circular cross-section)
Bore 116: diameter/length: 0.5 mm/4 mm (circular cross-section)
Bore 118: diameter/length: 0.5 mm/4 mm (circular cross-section)
Microchannel 124: cross-sectiorial shape/width/depth/length/cross-sectional area: rectangular shape/350 µm/100 µm/12.5 mm/35,000 µm²
Microchannel 126: cross-sectional shape/width/depth/length/cross-sectional area: rectangular shape /50 µm/100 µm/10 mm/5,000 µm²
Bore 130: diameter/length: 500 µm/10 mm (circular cross-section)

The size of the microchannels (124 and 126 in Fig. 1) at which the aqueous phase and the organic solvent phase are collided is defined in the preferable range in context with flow rates of the aqueous phase and the organic solvent phase.

In the production method of the invention, it is preferable that, after emulsifying and dispersing, the water-soluble organic solvent having been used is removed via the microchannels. Examples of the methods of removing the solvent include an evaporation method using a rotary evaporator, a flash evaporator or an ultrasound atomizer, and a membrane separation method using an ultrafiltration membrane or a reverse osmosis membrane, and an ultrafiltration membrane method is particularly preferable.

An ultra filter (UF) is an apparatus in which a stock solution (an aqueous solution that is a mixture of water, a high-molecular substance, a low-molecular substance, a colloidal substance, and the like) having been pressurized is injected, separated into two types of solutions, namely, a filtrate (the low-molecular substance) and a concentrate (the high-molecular substance or colloidal substance) and then taken out.

An ultrafiltration membrane is a typical asymmetric membrane produced by the Loeb-Sourirajan method. Examples of polymer materials that can be used for ultrafiltration membrane include polyacrylonitrile, polyvinyl chloride-polyacrylonitrile copolymer, polysulfone, polyether sulfone, vinylidene fluoride, aromatic polyamide, cellulose acetate and the like. In recent years, ceramic membranes have also been employed. Different from the reverse osmosis method and the like, no pre-treatment is performed in the ultrafiltration method, which causes fouling, that is, the deposited of the polymers or the like on the membrane surface. Therefore, it is a common to practice to wash the membrane with a chemical or hot water at regular intervals. Thus, the membrane material should be resistant to chemicals and heat. There are carious membrane modules types of an ultrafiltration membrane such as a flat membrane type, a tubular type, a hollow fiber type and a spiral type. The performance of an ultrafiltration membrane is indicated in a molecular weight cut off, and various membranes having a molecular weight cut off of from 1,000 to 300,000 are commercially available. Examples of the commercially available membrane modules include, but are not limited to, MICROSA UF (Asahi Kasei Chemicals Corporation), capillary-type element NTU-3306 (Nitto Denko Corporation) and the like.

To remove the solvent from the emulsion of the present invention, the material of a membrane is particularly preferably polysulfone, polyether sulfone or aromatic polyamide, from the viewpoint of resistance for solvents. With respect to the membrane module form, flat membranes are mainly employed on the laboratory scale, while membranes of the hollow fiber type and spiral type are employed industrially. In particular, hollow fiber type membranes are preferable. Although the molecular weight cut off varies depending on the kind of the active ingredient, membranes having molecular weight cut off of in the range of from 5,000 to 100,000 are commonly used.
Although the available operation temperature is from 0°C to 80°C, a temperature range of 10°C to 40°C is particularly preferable in view of degradation of the active ingredient.

Examples of the laboratory-scale ultrafiltration apparatuses include flat membrane-type module ADVANTEC-UHP (ADVANTEC), flow-type labo-test unit RUM-2 (Nitto Denko Corporation), and the like. An industrial plant can be constructed by combining the individual membrane modules in any number and size to satisfy the required depending capacity. As a bench-scale unit, RUW-5A (Nitto Denko Corporation) and the like are commercially available.

In the production method of the invention, it is preferable to concentrate the emulsion after removing the solvent. For the concentration, the same method and device as those described with respect to removing the solvent, that is, the evaporation method, the filtration method or the like, and the devices for these methods, can be used. In the concentration, it is also preferable to employ the ultra-filtration method. Although it is preferable to use, if possible, the same membrane as in the solvent removal, ultrafiltration membranes having a different molecular weight cut off may be used if required. It is also possible to conduct the concentration at a different temperature from the solvent removal so as to elevate the concentration efficiency.

The emulsion obtained by the production method according to the present invention is an oil-in-water type emulsion. From the viewpoint of the transparency of the obtained emulsion, the volume-average particle diameter (median diameter) of the oil droplets in the emulsion is preferably 200 nm or less, more preferably from 1 nm to 100 nm, and still more preferably from 1 nm to 50 nm.
The aqueous dispersion a solid obtained by the production method according to the invention is an aqueous solid dispersion. From the viewpoint of the transparency of the obtained dispersion, the volume-average particle diameter (median diameter) of the hydrophobic solid microparticles (dispersed particles) is preferably 200 nm or less, more preferably from 1 nm to 100 nm, and still more preferably from I nm to 50 nm.

The particle diameter of the emulsified or dispersed particles of the invention can be measured with a commercially available particle diameter distribution measuring device or the like. Known examples of the method of measuring the particle diameter distribution include a optical microscopy method, a confocal laser microscopy method, an electron microscopy method, an atomic force microscopy method, a static light scattering method, a laser diffraction method, a dynamic light scattering method, a centrifugal precipitation method, an electric pulse measurement method, a chromatography method, an ultrasonic damping method and the like, and devices corresponding to the respective principle are commercially available.

In consideration of the volume-average particle diameter range in the invention and ease of measurement, the dynamic light scattering method is preferable for measuring the volume-average particle diameter of the emulsion or dispersion of the invention. Examples of the commercially available measurement devices using dynamic light scattering include NANOTRAC UPA (Nikkiso Co., Ltd.), a dynamic light scattering particle diameter distribution measuring device LB-550 (Horiba, Ltd.), a thick type particle diameter analyzer FPAR-1000 (Otsuka Electronics Co., Ltd.) and the like.
In the invention, the volume-average particle diameter is measured by using the dynamic light scattering particle diameter distribution measuring device at 25°C.

The emulsion or dispersion obtained according to the invention is a fine emulsion or dispersion in which deterioration of the quality of the natural ingredient is remarkably reduced. Therefore, the emulsion or dispersion is favorably used for various applications depending on the types of the natural ingredients.
Examples of the applications include foods, skin external preparations, and drugs.
That is, a food according to the invention is a food containing an emulsion or dispersion obtained according to the production method described above, and the natural ingredient is a functional food ingredient. The functional food ingredient may be any of the natural ingredients described above that is usable in food applications, and specific examples thereof include, but are not limited to, terpenoids and polyphenols.
A skin external preparation according to the invention is a skin external preparation containing an emulsion or dispersion obtained according to the above production method, wherein the natural ingredient described above is a skin external preparation ingredient. The skin external preparation ingredient may be any of the above-described natural ingredients that is usable in skin external applications, and specific examples thereof include, but are not limited to, fatty acids, complex lipids, and terpenoids.
A drug according to the invention is a drug containing an emulsion or dispersion obtained according to the above production method, wherein the natural ingredient is a pharmaceutical ingredient. The pharmaceutical ingredient may be any of the above-described natural ingredients that is usable in pharmaceutical applications, and specific examples thereof include, but are not limited to, alkaloids and steroids.

### EXAMPLES

The present invention is described more specifically below by reference to examples. In the description below, "part(s)" and "%" are based on mass unless indicated otherwise.

### Example 1

### (Preparation of Emulsion A)

280g of pure water was prepared as a water phase A.
Further, the following components were dissolved at 65°C for 1 hour, and thereafter cooled to 25°C, whereby an oil phase A was obtained.
- Haematococcus algae extract (content of astaxanthins: 10% by mass): 0.6 g
- Mixed tocopherol: 0.1 g
- Sucrose stearate (HLB = 15): 0.3 g
- decaglyceryl monooleate (HLB = 12): 0.3 g
- Ethanol: 38.4 g

Here, the sucrose stearate used was RYOTO Sugar Ester S-1670 (HLB = 15) manufactured by Mitsubishi-Kagaku Foods Corp., and the decaglyceryl monooleate used was NIKKOL DECAGLYN 1-O (HLB-12) manufactured by Nikko Chemicals Co. Ltd. The Haematococcus algae extract used was ASTOTS-10O manufactured by Takeda Shiki Co., Ltd. The Haematococcus algae extract was a product in the form of an isolated dried oily material. The mixed tocopherol used was REKEN E OIL 800 manufactured by Riken Vitamin Co., Ltd. The ethanol used was a special grade reagent manufactured by Wako Pure Chemical Industries Ltd.

A slit-interdigital SSIMM-SS-Ni25 (manufactured by IMM Gmbh), in which 5µm sintered metal filters were provided at respective flow channels, was prepared as the micromixer to be used for emulsification, and all of the micromixer, the aqueous phase A, and the oil phase A were placed in an environment of 25°C.
A precision metering pump was set to provide an aqueous phase flow rate of 21.0 ml/min and an oil phase flow rate of 3.0 ml/min, and the aqueous phase and the oil phase were respectively introduced into the micromixer to perform micro-mixing.
Subsequently, an emulsion was sampled when the flow rates and the liquid feeding pressures became stable, and the emulsion is referred to as astaxanthin-containing emulsion A. The particle diameters of the oil droplets of the emulsion A were measured with a FPAR1000 particle size analyzer manufactured by manufactured by Otsuka Electronics Co., Ltd., and the median diameter thereof was found to be 135 nm.

### (Preparation of Emulsion B)

The compositions of the aqueous phase and the oil phase, and the preparation methods were exactly the same as in the case of the emulsion A; however, a KM-type micromixer 100/100, which is a counter-collision micromixer, was used as the micromixer used for emulsification. In this micromixer, the microchannels for pre-collision liquids, i.e., the microchannels 124 and the microchannels 126 in Fig. 1, have rectangular shapes and the following sizes:
Microchannel 124: width/depth/length: 100µm/100µm/12.5mm
Microchannel 126: width/depth/length: 100µm/100µm/10mm
Emulsification was performed in the same manner as in the case of the emulsion A, except that this micromixer was used for the emulsification while introducing the aqueous phase to the outer annular channel at a flow rate of 21.0 ml/min and introducing the oil phase to the inner annular channel at a flow rate of 3.0 ml/min. As a result, an emulsion B was obtained. The median diameter of the oil droplets of the emulsion B was 45 nm.

### (Preparation of Emulsion C)

The compositions of the aqueous phase and the oil phase, and the preparation methods were exactly the same as in the case of the emulsion A; however, a KM-type micromixer 350/50 was used as the micromixer used for emulsification. In this micromixer, the microchannels for pre-collision liquids, i.e., the microchannels 124 and the microchannels 126 in Fig. 1, have rectangular shapes and the following sizes:
Microchannel 124: width/depth/length: 350 µm/100µm/12.5mm
Microchannel 126: width/depth/length: 50 µm/100µm/10mm
Emulsification was performed in the same manner as in the case of the emulsion A, except that this micromixer was used for the emulsification while introducing the aqueous phase to the outer annular channel (microchannels 124) at a flow rate of 42.0 ml/min and introducing the oil phase to the inner annular channel (microchannels 126) at a flow rate of 6.0 ml/min. As a result, an emulsion C was obtained. The median diameter of the oil droplets of the emulsion C was 19 nm.

### (Preparation of Emulsion D)

The compositions of the aqueous phase and the oil phase, and the preparation methods were exactly the same as in the case of the emulsion C, and the micromixer used for emulsification was the KM-type micromixer 350/50. However, unlike the emulsion C, the flow rate of the aqueous phase introduced to the inner annular channel (microchannels 126) was 42.0 ml/min, and the flow rate of the oil phase introduced to the outer annular channel (microflow channels 124) was 6.0 ml/min. Except for these points, emulsification was performed in the same manner as in the case of the emulsion C, whereby an emulsion D was obtained. The median diameter of the oil droplets of the emulsion D was 95 nm.

### (Preparation of Emulsion E)

The compositions of the aqueous phase and the oil phase, and the preparation methods were exactly the same as in the case of the emulsion A; however, a KM-type micromixer 200/100 was used as the micromixer used for emulsification. In this micromixer, the microchannels for pre-collision liquids, i.e., the microchannels 124 and the microchannels 126 in Fig. 1, have rectangular shapes and the following sizes:
Microchannel 124: width/depth/length: 200 µm/100µm/12.5mm
Microchannel 126: width/depth/length: 100 µm/100µm/10mm
Emulsification was performed in the same manner as in the case of the emulsion A, except that this micromixer was used for the emulsification while introducing the aqueous phase to the outer annular channel (microchannels 124) at a flow rate of 31.5 ml/min and introducing the oil phase to the inner annular channel (microchannels 126) at a flow rate of 4.5 ml/min. As a result, an emulsion E was obtained. The median diameter of the oil droplets of the emulsion E was 28 nm.

### (Preparation of Emulsion F)

The compositions of the aqueous phase and the oil phase, and the preparation methods were exactly the same as in the case of the emulsion E, and the micromixer used for emulsification was the KM-type micromixer 200/100. However, unlike the emulsion E, the flow rate of the aqueous phase introduced to the inner annular channel (microchannels 126) was 31.5 ml/min, and the flow rate of the oil phase introduced to the outer annular channel (microflow channels 124) was 4.5 ml/min. Except for these points, emulsification was performed in the same manner as in the case of the emulsion E, whereby an emulsion F was obtained. The median diameter of the oil droplets of the emulsion F was 55 nm.

### (Preparation of Emulsion G)

The aqueous phase A and the oil phase A were mixed with a stirrer so as to provide a mixture having the same composition as that of the emulsion A, and, immediately thereafter, emulsification was performed for 5 min using an agitation-type homogenizer (manufactured by Nippon Seiki Co., Ltd.) at 10000 rpm, as a result of which an astaxanthin-containing emulsion G was obtained. The median diameter of the oil droplets of the emulsion G was 347 nm.

### (Preparation of Emulsion H)

*Haematococcus pluvialis* alga containing astaxanthin was cooled to -50°C, and dehydrated by freeze drying. Then, sodium chloride was added thereto, and pulverization operation was performed. As a result, fine powder of the Haematococcus alga was obtained. The Haematococcus alga fine powder was immersed in ethanol in a refrigerator for 24 hours, so that astaxanthin was extracted from the Haematococcus alga.
Ethanol was added to the extract such that the absorbance of the extract liquid at 478 nm became the same as that of the oil phase A. The mixed tocopherol, the sucrose stearate, and the decaglyceryl monooleate were added to and dissolved in the obtained Haematococcus alga ethanol extract liquid such that the respective components have the same compositional ratios as those of the corresponding components in the oil phase A. As a result, an oil phase H was obtained.
The oil phase H and the aqueous phase A were subjected to micromixer emulsification in exactly the same manner as in the case of the emulsion A, whereby an emulsion H was obtained. The median diameter of the oil droplets of the emulsion H was 120 nm.

### (Preparation of Emulsion I)

Preparation was performed in the same manner as in the case of the emulsion B, except that the oil phase H was used in place of the oil phase A. The median diameter of the oil droplets of the emulsion I was 37 nm.

### (Preparation of Emulsion J)

Preparation was performed in the same manner as in the case of the emulsion C, except that the oil phase H was used in place of the oil phase A. The median diameter of the oil droplets of the emulsion J was 12 nm.

### (Preparation of Emulsion K)

Preparation was performed in the same manner as in the case of the emulsion D, except that the oil phase H was used in place of the oil phase A. The median diameter of the oil droplets of the emulsion K was 89 nm.

### (Preparation of Emulsion L)

Preparation was performed in the same manner as in the case of the emulsion E, except that the oil phase H was used in place of the oil phase A. The median diameter of the oil droplets of the emulsion L was 26 nm.

### (Preparation of Emulsion M)

Preparation was performed in the same manner as in the case of the emulsion F, except that the oil phase H was used in place of the oil phase A. The median diameter of the oil droplets of the emulsion M was 54 nm.

### (Preparation of Emulsion N)

Preparation was performed in the same manner as in the case of the emulsion G, except that the oil phase H was used in place of the oil phase A. The median diameter of the oil droplets of the emulsion N was 330 nm.

Each of the emulsions A to N were processed using a laboratory-scale ultrafiltration apparatus ADVANTEC-UHP-43K with an ultrafiltration membrane Q0500043E (50000Da) made of polysulfone, thereby removing ethanol to a content of 0.1%; further, concentration operation was performed using the same apparatus, thereby concentrating the emulsion until the astaxanthin content became 1.0%. As a result, concentrated emulsions A to N were obtained.

Each of the concentrated emulsions A to N were evaluated by ten people, including men and women, with respect to the presence or absence of odor, and was graded - (acceptable level), + (somewhat uncomfortable level), and ++ (problematic level).
Each of the emulsions was stored at 50°C for 21 days, and a residual colorant ratio was measured. The residual colorant ratio was determined by measuring, using a spectrophotometer, the absorbance of a liquid that was obtained by diluting the emulsion before storage 3,000 times with pure water and the absorbance of a liquid that was obtained by diluting the emulsion after storage 3,000 times with pure water, and obtaining a ratio of the absorbance at 478 nm after storage to the absorbance at 478 nm before storage.
The results with respect to the oil droplet diameter, the evaluation on odor, and the residual colorant ratio obtained by storage at 50°C for 21 days are summarized in Table 1.

**Table I**

| Sample | Preparation Conditions | | | | | Evaluation Results | | |
|---|---|---|---|---|---|---|---|---|
| | Between Extraction and Emulsification | Emulsification Apparatus | Oil Phase Microflow Channel Cross-sectional Area (µm²) | Aqueous Phase Microflow Channel Cross-sectional Area (µm²) | Vo/Vw | Oil Droplet Median Diameter (nm) | Odor | Residual Colorant Ratio After 50°C for 21 days (%) |
| A | Once Dried | Slit-interdigital micro | 625 | 625 | 0.14 | 135 | + | 85 |
| B | Once Dried | Counter-collision micro | 10000 | 10000 | 0.14 | 45 | + | 76 |
| C | Once Dried | Counter-collision micro | 5000 | 35000 | 1.00 | 19 | + | 62 |
| D | Once Dried | Counter-collision micro | 35000 | 5000 | 0.02 | 95 | + | 83 |
| E | Once Dried | Counter-collision micro | 10000 | 20000 | 0.29 | 28 | + | 64 |
| F | Once Dried | Counter-collision micro | 20000 | 10000 | 0.07 | 55 | + | 81 |
| G | Once Dried | Stirring-type homogenizer | --- | --- | --- | 347 | ++ | 77 |
| H | Continuous | Slit-interdigital micro | 625 | 625 | 0.14 | 120 | - | 90 |
| I | Continuous | Counter-collision micro | 10000 | 10000 | 0.14 | 37 | - | 92 |
| J | Continuous | Counter-collision micro | 5000 | 35000 | 1.00 | 12 | - | 93 |
| K | Continuous | Counter-collision micro | 35000 | 5000 | 0.02 | 89 | - | 95 |
| L | Continuous | Counter-collision micro | 10000 | 20000 | 0.29 | 26 | - | 92 |
| M | Continuous | Counter-collision micro | 20000 | 10000 | 0.07 | 54 | - | 93 |
| N | Continuous | Stirring-type homogenizer | --- | --- | --- | 330 | + | 88 |

As shown in Fig. 1, use of a counter-collision micromixer enabled formation of drastically finer emulsion with high transparency, as compared to cases in which a stirring-type emulsification apparatus or a slit-interdigital micromixer was used. Emulsions B to F are reference examples, and emulsions I to M belong to the present invention.

Although the same astaxanthin extracted from Haematococcus algae were used, the emulsions B to F, the preparation of which includes once isolating the astaxanthin as a dried oily product and dissolving it in ethanol to form an oil phase, exhibited uncomfortable odor and inferior storage stability of the astaxanthin colorant when storing over time. In contrast, it was clearly demonstrated that the emulsions I to M, the oil phase of which was formed through a continuous process without drying and solidifying the liquid obtained by ethanol extraction of astaxanthin from Haematococcus algae, exhibited ameliorated odor and improved storage stability of the astaxanthin.

Further, when emulsification was performed using a once-dried oily astaxanthin as a raw material, there is a tendency for a smaller diameter of emulsion oil droplets to cause somewhat inferior temporal stability. However, when emulsification was performed using a raw material that had been prepared through a continuous process without drying and solidifying the extract liquid, a further decrease in emulsion oil droplet diameter was enabled while maintaining the temporal stability of the astaxanthin at a favorable level (the emulsions I to M).

As demonstrated above, a fine emulsion or dispersion of a natural ingredient extracted from an animal or plant by using a water-soluble organic solvent is provided with extremely small deterioration of the quality of the natural ingredient, according to the invention.

## Claims

1. A method of producing an emulsion or dispersion, comprising the subsequent steps of:
(i) passing an aqueous solution (w) and a water-soluble organic solvent solution (o) containing a natural ingredient that has been extracted from a natural animal or plant using a water-soluble organic solvent through respective microflow channels each of which has a cross-sectional area at the narrowest portion of 1 µm² to 1 mm²; and
(ii) mixing the solutions (w) and (o) by counter collision.

2. The method of claim 1, wherein the volume average particle diameter of oil droplet particles or dispersed particles in the obtained emulsion or dispersion is 1-100 nm, and preferably 1-50 nm.

3. The method of claim 1 or 2, wherein the ratio (Vo/Vw) of a flow speed (Vo) of solution (o) to a flow speed (Vw) of the aqueous solution (w) is 0.05-5 during the mixing.

4. The method of any of claims 1-3, wherein the flow rate of solution (o) is 1-70 ml/min.

5. The method of any of claims 1-4, wherein the flow rate of solution (w) is 10-500 ml/min.

6. The method of any of claims 1-5, further comprising after step (ii) removing the water-soluble organic solvent from the obtained emulsion or dispersion.

7. The method of any of claims 1-6, wherein solution (o) contains at least one nonionic surfactant having an HLB value of 10-16.

8. The method of any of claims 1-7, wherein the water-soluble organic solvent is ethanol or a mixture of ethanol and water.

9. The method of any of claims 1-8, wherein the natural ingredient is a functional food ingredient.

10. The method of any of claims 1-8, wherein the natural ingredient is a skin external preparation ingredient.

11. The method of any of claims 1-8, wherein the natural ingredient is a pharmaceutical ingredient.

12. A food comprising an emulsion or dispersion produced by the method of claim 9, wherein the volume average particle diameter of oil droplet particles or dispersed particles in the obtained emulsion or dispersion is 1-50 nm.

13. A skin external preparation comprising an emulsion or dispersion produced by the method of claim 10, wherein the volume average particle diameter of oil droplet particles or dispersed particles in the obtained emulsion or dispersion is 1-50 nm.

14. A drug comprising an emulsion or dispersion produced by the method of claim 11 wherein the volume average particle diameter of oil droplet particles or dispersed particles in the obtained emulsion or dispersion is 1-50 nm.

## Patentansprüche

1. Verfahren zur Herstellung einer Emulsion oder Dispersion, umfassend die aufeinanderfolgenden Schritte:
(i) Hindurchführen einer wässrigen Lösung (w) und einer Lösung eines wasserlöslichen organischen Lösungsmittels (o), enthaltend einen natürlichen Inhaltsstoff, der aus einem natürlichen Tier oder einer Pflanze unter Verwendung eines wasserlöslichen organischen Lösungsmittels extrahiert worden ist, durch jeweilige Mikrofluss-Leitungen, von denen jede eine Querschnittsfläche am engsten Teil von 1 µm² bis 1 mm² aufweist; und
(ii) Mischen der Lösungen (w) und (o) durch Gegenkollision.

2. Verfahren gemäß Anspruch 1, worin der volumengemittelte Partikeldurchmesser von Öltröpfchenpartikeln oder dispergierten Partikeln in der erhaltenen Emulsion oder Dispersion 1 bis 100 nm, und bevorzugt 1 bis 50 nm, beträgt.

3. Verfahren gemäß Anspruch 1 oder 2, worin das Verhältnis (Vo/Vw) der Fließgeschwindigkeit (Vo) der Lösung (o) zu der Fließgeschwindigkeit (Vw) der wässrigen Lösung (w) während des Mischens 0,05 bis 5 beträgt.

4. Verfahren gemäß irgendeinem der Ansprüche 1 bis 3, worin die Flussrate der Lösung (o) 1 bis 70 ml/min beträgt.

5. Verfahren gemäß irgendeinem der Ansprüche 1 bis 4, worin die Flussrate der Lösung (w) 10 bis 500 ml/min beträgt.

6. Verfahren gemäß irgendeinem der Ansprüche 1 bis 5, ferner umfassend, nach dem Schritt (ii), das Entfernen des wasserlöslichen organischen Lösungsmittels von der erhaltenen Emulsion oder Dispersion.

7. Verfahren gemäß irgendeinem der Ansprüche 1 bis 6, worin die Lösung (o) mindestens ein nicht-ionisches Tensid mit einem HLB-Wert von 10 bis 16 enthält.

8. Verfahren gemäß irgendeinem der Ansprüche 1 bis 7, worin das wasserlösliche organische Lösungsmittel Ethanol oder eine Mischung von Ethanol und Wasser ist.

9. Verfahren gemäß irgendeinem der Ansprüche 1 bis 8, worin der natürliche Inhaltsstoff ein funktioneller Nahrungsmittel-Inhaltsstoff ist.

10. Verfahren gemäß irgendeinem der Ansprüche 1 bis 8, worin der natürliche Inhaltsstoff ein Inhaltsstoff für eine externe Hautzubereitung ist.

11. Verfahren gemäß irgendeinem der Ansprüche 1 bis 8, worin der natürliche Inhaltsstoff ein pharmazeutischer Inhaltsstoff ist.

12. Nahrungsmittel, umfassend eine Emulsion oder Dispersion, hergestellt durch das Verfahren gemäß Anspruch 9, worin der volumengemittelte Partikeldurchmesser von Öltröpfchenpartikeln oder dispergierten Partikeln in der erhaltenen Emulsion oder Dispersion 1 bis 50 nm beträgt.

13. Externe Hautzubereitung, umfassend eine Emulsion oder Dispersion, hergestellt durch das Verfahren gemäß Anspruch 10, worin der volumengemittelte Partikeldurchmesser von Öltröpfchenpartikeln oder dispergierten Partikeln in der erhaltenen Emulsion oder Dispersion 1 bis 50 nm beträgt.

14. Arzneimittel, umfassend eine Emulsion oder Dispersion, hergestellt durch das Verfahren gemäß Anspruch 11, worin der volumengemittelte Partikeldurchmesser von Öltröpfchenpartikeln oder dispergierten Partikeln in der erhaltenen Emulsion oder Dispersion 1 bis 50 nm beträgt.

## Revendications

1. Procédé de production d'une émulsion ou d'une dispersion, comprenant les étapes suivantes consistant :
(i) à faire passer une solution aqueuse (w) et une solution de solvant organique soluble dans l'eau (o) contenant un ingrédient naturel qui a été extrait d'un animal ou d'une plante naturelle en utilisant un solvant organique soluble dans l'eau par des canaux de micro-écoulement respectifs dont chacun présente une section transversale à la portion la plus étroite de 1 µm² à 1 mm² ; et
(ii) à mélanger les solutions (w) et (o) par contre collision.

2. Procédé selon la revendication 1, dans lequel le diamètre moyen de particule en volume des particules de gouttelettes ou des particules dispersées d'huile dans l'émulsion ou la dispersion obtenue est de 1-100 nm, et de préférence de 1-50 nm.

3. Procédé selon la revendication 1 ou 2, dans lequel le rapport (Vo/Vw) d'une vitesse d'écoulement (Vo) de la solution (o) à une vitesse d'écoulement (Vw) de la solution aqueuse (w) est de 0,05-5 pendant le mélange.

4. Procédé selon l'une quelconque des revendications 1-3, dans lequel le débit de la solution (o) est de 1-70 ml/min.

5. Procédé selon l'une quelconque des revendications 1-4, dans lequel le débit de la solution (w) est de 10-500 ml/min.

6. Procédé selon l'une quelconque des revendications 1-5, comprenant de plus après l'étape (ii) l'élimination du solvant organique soluble dans l'eau de l'émulsion ou de la dispersion obtenue.

7. Procédé selon l'une quelconque des revendications 1-6, dans lequel la solution (o) contient au moins un tensioactif non ionique ayant une valeur HLB de 10-16.

8. Procédé selon l'une quelconque des revendications 1-7, dans lequel le solvant organique soluble dans l'eau est l'éthanol ou un mélange d'éthanol et d'eau.

9. Procédé selon l'une quelconque des revendications 1-8, dans lequel l'ingrédient naturel et un ingrédient d'aliment fonctionnel.

10. Procédé selon l'une quelconque des revendications 1-8, dans lequel l'ingrédient naturel est un ingrédient pour une préparation externe pour la peau.

11. Procédé selon l'une quelconque des revendications 1-8, dans lequel l'ingrédient naturel est un ingrédient pharmaceutique.

12. Aliment comprenant une émulsion ou une dispersion produite par le procédé selon la revendication 9, dans lequel le diamètre moyen de particule en volume de particules de gouttelettes ou de particules dispersées d'huile dans l'émulsion ou la dispersion obtenue est de 1-50 nm.

13. Préparation externe pour la peau comprenant une émulsion ou une dispersion produite par le procédé selon la revendication 10, dans laquelle le diamètre moyen de particule en volume de particules de gouttelettes ou de particules dispersées d'huile dans l'émulsion ou la dispersion obtenue est de 1-50 nm.

14. Médicament comprenant une émulsion ou une dispersion produite par le procédé selon la revendication 11, dans lequel le diamètre moyen de particule en volume de particules de gouttelettes ou de particules dispersées d'huile dans l'émulsion ou la dispersion obtenue est de 1-50 nm.
